# EUROPEAN PATENT SPECIFICATION

(11) **EP 4 157 434 B1**
(45) Date of publication and mention of the grant of the patent: **16.09.2026**
(21) Application number: 21831539.8
(22) Date of filing: 01.07.2021
(51) Int. Cl.: G06F 3/01, A61B 5/00, A61B 5/293, A61N 1/04, A61N 1/05, A61N 1/36, A61M 5/172

(54) **SYSTEMS AND METHODS DECODING INTENDED SYMBOLS FROM NEURAL ACTIVITY**
SYSTEME UND VERFAHREN ZUR DECODIERUNG VON BEABSICHTIGTEN SYMBOLEN AUS NEURONALER AKTIVITÄT
SYSTÈMES ET PROCÉDÉS DE DÉCODAGE DE SYMBOLES CHOISIS À PARTIR D'UNE ACTIVITÉ NEURONALE

(30) Priority: 01.07.2020 US 202063047196 P; 28.08.2020 US 202017006645
(43) Date of publication of application: 05.04.2023
(73) Proprietor: The Board of Trustees of the Leland Stanford Junior University, Stanford, CA 94305-2038 (US)
(72) Inventor: SHENOY, Krishna, V., Stanford, CA 94305 (US); HENDERSON, Jaimie, M., Stanford, CA 94305 (US); WILLETT, Francis Robert, Stanford, CA 94305-2038 (US)
(74) Representative: Auger, Hannah Maria
(86) International application number: PCT/US2021/040192
(87) International publication number: WO 2022/006462

(56) References cited:
- US-A1- 2006 159 195
- US-A1- 2012 221 075
- US-A1- 2013 253 299
- US-A1- 2014 358 192
- US-A1- 2016 180 215
- US-A1- 2016 299 568
- US-A1- 2017 108 926
- US-A1- 2017 147 077
- US-A1- 2019 333 505
- US-A1- 2020 142 481
- FRANCIS R. WILLETT: "High-performance brain-to-text communication via imagined handwriting", BIORXIV, 2 July 2020 (2020-07-02), XP093154080, Retrieved from the Internet <URL:https://www.biorxiv.org/content/10.1101/2020.07.01.183384v1.full.pdf> [retrieved on 20240422], DOI: 10.1101/2020.07.01.183384
- FRANCIS R. WILLETT: "High-performance brain-to-text communication via imagined handwriting - Supplementary Materials", BIORXIV, 2 July 2020 (2020-07-02), pages 1 - 20, XP093154678, Retrieved from the Internet <URL:https://www.biorxiv.org/content/biorxiv/early/2020/07/02/2020.07.01.183384/DC2/embed/media-2.pdf> [retrieved on 20240422], DOI: 10.1101/2020.07.01.183384
- TADANOBU MISAWA ET AL: "A Single-Trial Multiclass Classification of Various Motor Imagery Tasks for EEG-Based Brain-Computer Interface Communication", ELECTRONICS AND COMMUNICATIONS IN JAPAN, WILEY-BLACKWELL PUBLISHING, INC, US, vol. 100, no. 1, 13 December 2016 (2016-12-13), pages 18 - 26, XP072447393, ISSN: 1942-9533, DOI: 10.1002/ECJ.11916
- SPEIER W ET AL: "Integrating language models into classifiers for BCI communication: a review", JOURNAL OF NEURAL ENGINEERING, INSTITUTE OF PHYSICS PUBLISHING, BRISTOL, GB, vol. 13, no. 3, 6 May 2016 (2016-05-06), pages 31002, XP020304661, ISSN: 1741-2552, [retrieved on 20160506], DOI: 10.1088/1741-2560/13/3/031002
- BRANCO MARIANA P.; FREUDENBURG ZACHARY V.; AARNOUTSE ERIK J.; BLEICHNER MARTIN G.; VANSTEENSEL MARISKA J.; RAMSEY NICK F.: "Decoding hand gestures from primary somatosensory cortex using high-density ECoG", NEUROIMAGE, ELSEVIER, AMSTERDAM, NL, vol. 147, 5 December 2016 (2016-12-05), AMSTERDAM, NL , pages 130 - 142, XP029914427, ISSN: 1053-8119, DOI: 10.1016/j.neuroimage.2016.12.004
- BRUMBERG, J.S. ; NIETO-CASTANON, A. ; KENNEDY, P.R. ; GUENTHER, F.H.: "Braincomputer interfaces for speech communication", SPEECH COMMUNICATION, ELSEVIER SCIENCE PUBLISHERS , AMSTERDAM, NL, vol. 52, no. 4, 1 April 2010 (2010-04-01), NL , pages 367 - 379, XP026917078, ISSN: 0167-6393, DOI: 10.1016/j.specom.2010.01.001

## Description

### STATEMENT OF FEDERALLY SPONSORED RESEARCH

This invention was made with Government support under contract DC014034 awarded by the National Institutes of Health. The Government has certain rights in the invention.

### FIELD OF THE INVENTION

The present invention generally relates to decoding handwriting from neural activity.

### BACKGROUND

The human brain is a highly complex organ that generates thought and controls motor function of the body. These two functions are closely linked. Handwriting is generally the process of intending to write a specific glyph and performing the necessary motor actions to in fact write the glyph, e.g. by controlling the arm and hand to grip a pencil and draw the glyph on a piece of paper.

Neural signals in the brain can be recorded using a variety of methods that have different advantages and disadvantages. For example, electroencephalograms (EEGs) are useful for measuring local field potentials which measure average neural activity over a region. Smaller electrode arrays, such as (but not limited to) the Utah array, can be used to record the activity of a specific or small group of specific neurons.

US 2019/0333505 A1 discloses systems and methods for decoding intended speech from neuronal activity**.** One embodiment includes a neuronal speech system for decoding intended speech from neuronal signals comprising a neuronal signal recorder implanted into a user's brain, including a multielectrode array, controller circuitry, and a communication circuitry capable of transmitting data to a neuronal signal decoder.

Tadanobu Misawa et al.: "A single-trial multiclass classification of various motor imagery tasks for EEG-based brain-computer interface communication", Electronics and Communications in Japan (DOI: 10.1002/ECJ.11916), studies brain activity with various motor imagery tasks for improvement of brain-computer interface (BCI) usability using 14 electroencephalography (EEG) electrodes in five healthy subjects. The motor imagery tasks include three classical and one Kanji writing motor imagery tasks.

### SUMMARY OF THE INVENTION

The claims defined the subject matter for protection. Systems and methods for decoding intended symbols from neural activity in accordance with embodiments of the invention are illustrated.

One embodiment of the present disclosure includes a symbol decoding system for brain-computer interfacing, including a neural signal recorder implanted into a brain of a user, and a symbol decoder, the symbol decoder including a processor, and a memory, where the memory includes a symbol decoding application capable of directing the processor to obtain neural signal data from the neural signal recorder, estimate a symbol from the neural signal data using a symbol model, and perform a command associated with the symbol.

In another embodiment, the neural signal recorder is a microelectrode array including a plurality of electrodes.

In a further embodiment, the neural signal data describes spikes of neurons in proximity to respective electrodes in the plurality of electrodes.

In still another embodiment, the system further includes at least one output device.

In a still further embodiment, the output device is selected from the group consisting of vocalizers, displays, prosthetics, and computer systems.

In yet another embodiment, the symbol model is selected from the group consisting of: recurrent neural networks (RNNs), long short-term memory (LSTM) networks, temporal convolutional networks, and hidden Markov models (HMMs).

In a yet further embodiment, the symbol model is a recurrent neural network (RNN), and to estimate the symbol from the neural signal data, the symbol decoding application further directs the processor to temporally bin the neural signal data to create at least one neural population time series, convert the at least one neural population time series into at least one time probability series, and identify a most likely symbol from the at least one time probability series after a time delay triggered by identification of a high probability of a new character in the at least one time probability series.

In another additional embodiment, the memory further includes a symbol database including a plurality of symbols, and a plurality of commands, wherein each symbol in the plurality of symbols is associated with a command.

In a further additional embodiment, the plurality of symbols includes letters of an alphabet, and each letter of the alphabet is associated with a command to print the letter to a text string.

In another embodiment again, the text string is vocalized.

In a further embodiment again, the symbols for each letter in the alphabet are difference maximized.

In still yet another embodiment, a method for decoding symbols from neural activity includes obtaining neural signal data from a neural signal recorder implanted into a brain of a user, estimating a symbol from the neural signal data using a symbol model, and perform a command associated with the symbol using a symbol decoder.

In a still yet further embodiment, the neural signal recorder is a microelectrode array including a plurality of electrodes.

In still another additional embodiment, the neural signal data describes spikes of neurons in proximity to respective electrodes in the plurality of electrodes.

In a still further additional embodiment, the method further includes performing the command using at least one output device.

In still another embodiment again, the output device is selected from the group consisting of: vocalizers, displays, prosthetics, and computer systems.

In a still further embodiment again, the symbol model is selected from the group consisting of: recurrent neural networks (RNNs), long short-term memory (LSTM) networks, temporal convolutional networks, and hidden Markov models (HMMs).

In yet another additional embodiment, the symbol model is a recurrent neural network (RNN), and estimating the symbol from the neural signal data includes temporally binning the neural signal data to create at least one neural population time series, converting the at least one neural population time series into at least one time probability series, and identifying a most likely symbol from the at least one time probability series after a time delay triggered by identification of a high probability of a new character in the at least one time probability series

In a yet further additional embodiment, the symbol and the command are stored in a symbol database including a plurality of symbols, and a plurality of commands, wherein each symbol in the plurality of symbols is associated with a command.

In yet another embodiment again, the plurality of symbols includes letters of an alphabet, and each letter of the alphabet is associated with a command to print the letter to a text string.

In a yet further embodiment again, the symbols for each letter in the alphabet are difference maximized.

In another additional embodiment again, the text string is vocalized.

Additional embodiments and features are set forth in part in the description that follows, and in part will become apparent to those skilled in the art upon examination of the specification or may be learned by the practice of the invention. A further understanding of the nature and advantages of the present invention may be realized by reference to the remaining portions of the specification and the drawings, which forms a part of this disclosure.

### BRIEF DESCRIPTION OF THE DRAWINGS

The description and claims will be more fully understood with reference to the following figures and data graphs, which are presented as exemplary embodiments of the invention and should not be construed as a complete recitation of the scope of the invention.
FIG. 1 conceptually illustrates a glyph decoding system in accordance with an embodiment of the invention.
FIG. 2 is a block diagram conceptually illustrating a glyph decoder in accordance with an embodiment of the invention.
FIG. 3 is a flow chart illustrating a process for performing commands based on glyphs.

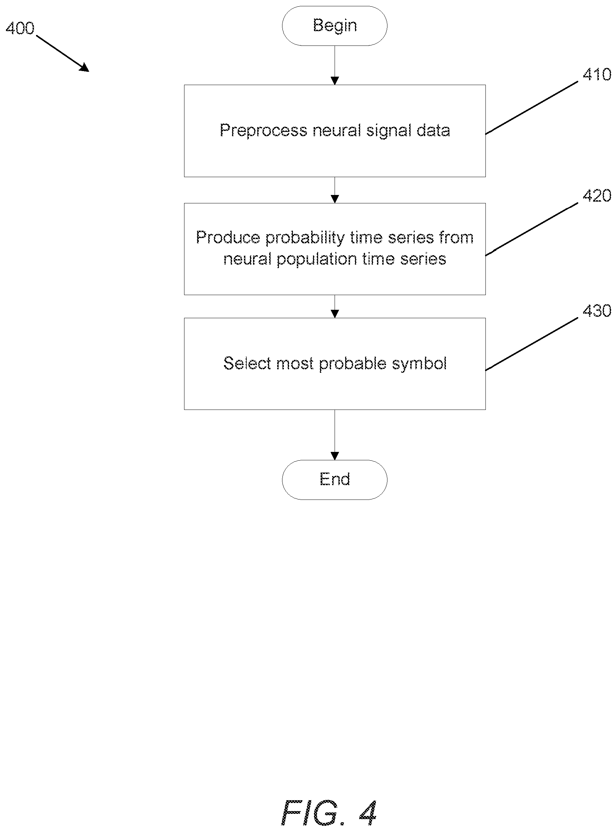

FIG. 4 is a flow chart illustrating a process for decoding neural signals into glyphs in accordance with an embodiment of the invention.
FIG. 5 is a flow chart for a process for generating training data for RNNs in accordance with an embodiment of the invention.
FIG. 6 is an example symbol space in accordance with an embodiment of the invention.

### DETAILED DESCRIPTION

Brain-computer interfaces (BCIs) are devices which turn neural activity in the brain into actionable, machine interpretable data. BCIs have many theoretical applications from control of prosthetic limbs to enabling users to type on a computer using only thought. Systems and methods described herein record neural signals from a user's brain and attempt to decode the signals into one of any number of symbols. In numerous embodiments, the neural signals that are recorded are related to brain activity triggered by the user imagining physically drawing the symbol. In many embodiments, the symbols are alphanumeric characters, and a user imagines handwriting the symbol. However, symbols can be any abstract shape, not restricted to any particular alphabet, as appropriate to the requirements of specific applications of embodiments of the invention. These symbols in turn can be associated with any number of commands. In many embodiments, an alphanumeric character can be associated with a command to print or register that alphanumeric character into a text string. Text strings can be vocalized or otherwise displayed to enable communication. In a variety of embodiments, arbitrary symbols can be assigned to specific functionality, e.g. a "spiral" shape may be used to trigger vocalization of a text string. However this is merely an example command and any arbitrary symbol can be associated with any arbitrary command.

Conventionally, BCI brain-to-text methodologies involve moving a digital cursor over a virtual keyboard where a user can "select" a key to enact that key's function, e.g. selecting a character. However, the act of moving the digital cursor is time consuming and limits the conversational speed of the user. Indeed, data collected suggests that timevarying patterns of movement, such as handwritten letters, can be decoded more easily compared to point-to-point movements. Instead of this more cumbersome interfacing, systems and methods described herein can directly translate imagined handwriting directly into functionality. That is, rather than simply moving a cursor, the user can imagine any arbitrary symbol which has been pre-assigned to a function. This elimination of the need for menus or keyboards rapidly improves the speed at which a user can interact with a computer. In many embodiments, the symbol space (e.g. the set of recognized symbols) is designed to maximize the distance between each symbol so that the timevarying patterns of movement are more easily discernable. In many embodiments, the distance between a pair of symbols is defined by the Frobenius norm of the difference between trajectory of an imagined writing stylus (e.g. a pen, pencil, other fanciful object) for each symbol. This can also be understood as maximizing the differences between two symbols. Using text input as an example, conventional BCI methods generally achieve at approximately 40 characters per minute, whereas systems and methods described herein have been used to achieve 90 characters per minute at greater than 99% accuracy with general purpose conventional autocorrect.

In order to achieve this functionality, systems and methods described herein associate particular neural signals to particular symbols. Turning now to the drawings, systems and methods for obtaining and decoding said neural signals into symbols in accordance with embodiments of the invention are described. System architectures for symbol decoding systems are described in further detail below.

### Symbol Decoding Systems

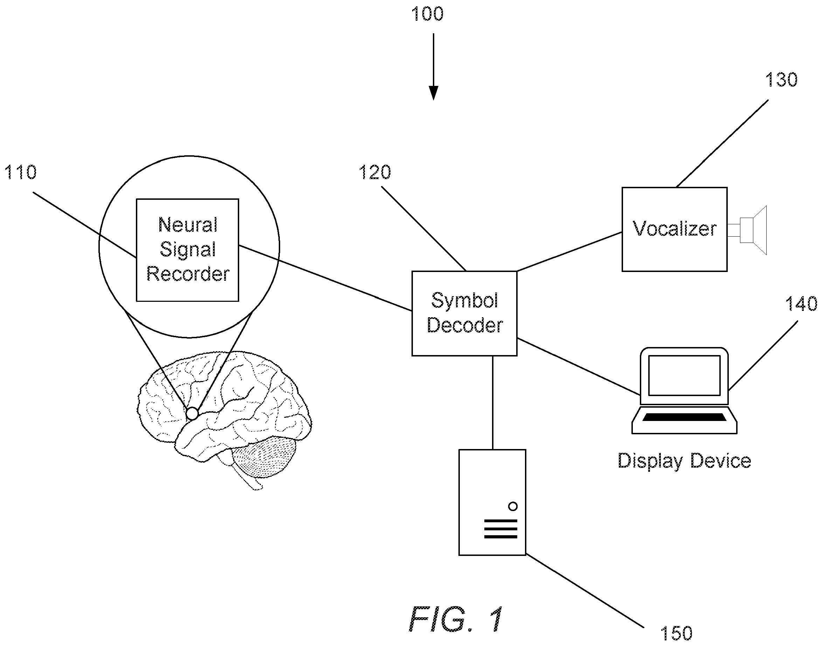

Symbol decoding systems can obtain neural signals from a brain using neural signal recorders, and decode the signals into symbols using symbol decoders. The decoded symbols in turn can be used to initiate any number of different commands. Turning now to FIG. 1, a system architecture for a symbol decoding system in accordance with an embodiment of the invention is illustrated.

Symbol decoding system 100 includes a neural signal recorder 110. In numerous embodiments, neural signal recorders are implantable microelectrode arrays such as (but not limited to) Utah arrays. The neural signal recorder can include transmission circuitry and/or any other circuitry required to obtain and transmit the neural signals. In many embodiments, the neural signal recorder is implanted into or sufficiently adjacent to the hand knob of the precentral gyrus. However, as one of ordinary skill in the art can appreciate, systems and methods described herein can implant the neural signal recorder into an number of different regions of the brain including (but not limited to) other motor regions, and focus signal acquisition and subsequent processing based on signals generated from that particular region. For example, instead of focusing on handwriting, similar systems and methods could focus on imagined movement of a leg in a particular fashion to produce similar results.

A symbol decoder 120 is in communication with the neural signal recorder. In numerous embodiments, symbol decoders are implemented using computer systems including (but not limited to) personal computers, server systems, cell phones, laptops, tablet computers, and/or any other computing device as appropriate to the requirements of specific applications of embodiments of the invention. The symbol decoder is capable of performing symbol decoding processes for interpreting the acquired neural signals and effecting the appropriate commands.

In many embodiments, the symbol decoder is connected to output devices which can be the subject of any of a number of different commands, including (but not limited to) vocalizer 130, display device 140, and computer system 150. In numerous embodiments, vocalizers can be used to read out text or provide other audio feedback to a user or the user's audience. Similarly, display devices can be used to visualize text or other graphics, and computing systems can generally perform any appropriate command for that computing system. As an example, a computing system may be able to send an email in accordance with commands input by the user. However as can be readily appreciated, any number of different computing systems can be used as an output device depending on the particular needs of the user and available set of commands.

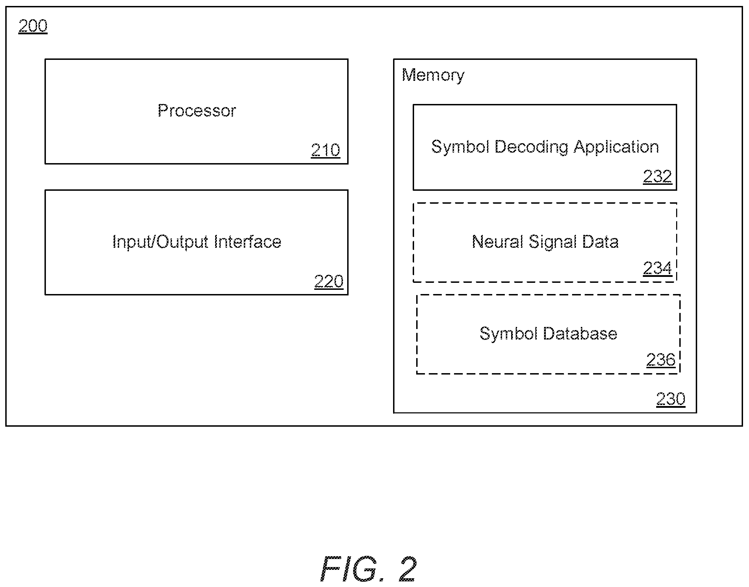

Symbol decoders, for example, can be constructed using any of a number of different computing devices. A block diagram for a symbol decoder in accordance with an embodiment of the invention is further illustrated in FIG. 2. Symbol decoder 200 includes a processor 210. Processors can be any number of one or more types of logic processing circuits including (but not limited to) central processing units (CPUs), graphics processing units (GPUs), field-programmable gate arrays (FPGAs), application-specific integrated circuits (ASICs), and/or any other logic circuit capable of carrying out symbol decoding processes as appropriate to the requirements of specific applications of embodiments of the invention.

The symbol decoder 200 further includes an input/output (I/O) interface 220. In numerous embodiments, I/O interfaces are capable of obtaining data from neural signal recorders. In various embodiments, I/O interfaces are capable of communicating with output devices and/or other computing devices. The symbol decoder 200 further includes a memory 230. The memory 230 contains a symbol decoding application 232. The symbol decoding application is capable of directing at least the processor to perform various symbol decoding processes such as (but not limited to) those described herein. In numerous embodiments, the symbol decoding application directs output devices to perform various commands.

In numerous embodiments, at various stages of operation the memory 230 contains neural signal data 324 and/or a symbol database 236. Neural signal data is data describing neuron activity in a user's brain recorded by the neural signal recorder. In many embodiments, the neural signal data reflects action potentials of individual or a small grouping of neurons (often referred to as "spikes") recorded using an electrode of an implanted microelectrode array. In a variety of embodiments, the neural signal data describes various spikes recorded at various different electrodes. Symbol databases can include any number of different symbols and associated commands. In many embodiments, different symbols can be associated with the same command. In various embodiments, the symbols are tailored to an individual user. E.g. a specific user may have a different variant of the letter "A" glyph as compared to a different user. Different symbol databases can be customized with different commands and symbols as desired by a user.

While particular system architectures and symbol decoders are discussed above with respect to FIGs. 1 and 2, any number of different architectures and symbol decoders can be used as appropriate to the requirements of specific applications of embodiments of the invention. For example, in numerous embodiments, a symbol decoding system may only have one output device, or various components may be wirelessly connected. As can be readily appreciated, many different implementations can be utilized without departing from the scope of the invention. Symbol decoding processes are discussed in further detail below.

### Symbol Decoding Processes

Symbol decoding processes can be used to translate brain activity of a user into specific, actionable commands in accordance to the user's intention. In many embodiments, the experience of the user can be akin to imagining drawing a symbol on a piece of paper using a pencil. A symbol decoding process in accordance with an embodiment of the invention can record the neural signals generated by the imagined writing and extract the symbol that was drawn on the imaginary piece of paper. That symbol can then be associated with a particular command such that any time the user imagines drawing that symbol, the command is executed.

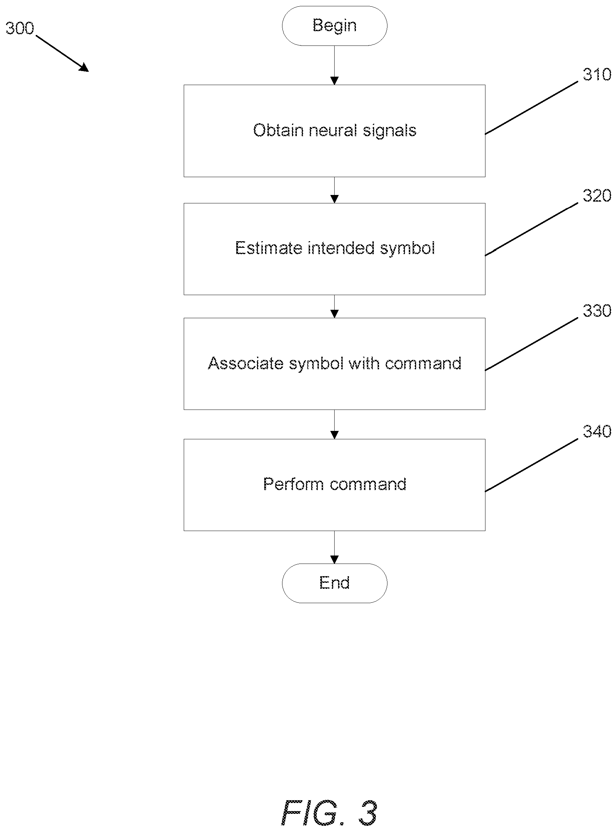

Turning now to FIG. 3, a high level flowchart for a symbol decoding process in accordance with an example is illustrated. Process 300 includes obtaining (310) neural signals from the user's brain while the user imagines physically drawing a symbol. It is important to note that the user does not need to physically move during the imagination process, and therefore those who cannot physically move a portion of their body can still utilize the systems and methods described herein. In numerous embodiments, the imagination process is specific to the user, and the user is recommended to imagine the same process each time. However, in numerous embodiments, deviation from an established imagined drawing can still yield satisfactory outcomes.

By way of example, in some embodiments, the user attempts and/or images drawing a symbol using a pencil on a piece of paper. However, some users may prefer to write in chalk on a slate. Indeed, any arbitrary scenario can be used so long as the user imagines and/or attempts physically drawing the symbol in a repeatable fashion. Indeed, any attempt at physical movement that traces a trajectory can be used as appropriate to the requirements of specific applications of embodiments of the invention. Further, depending on the location of the neural signal recorder in the brain, the drawing can be performed using the part of the body controlled by the region of the brain the neural signal recorder is in. For ease of description, the remainder of this description discusses in the context of imagined handwriting where the neural signal recorder records the hand knob of the precentral gyrus. As can be readily appreciated, any number of different imaginary writing scenarios can be used based on the location of the neural signal recorder as appropriate to the requirements of specific applications of embodiments of the invention. In many embodiments, the neural signals are recorded using a neural signal recorder as neural signal data.

The neural signal data is used to estimate (320) an intended symbol of the user. In many embodiments, the intended symbol is the one the user imagined drawing. Im various embodiments, the estimation is performed by a symbol model. Symbol models can infer underlying sequences of symbols from neural recordings. Symbol models are described in more detail in the following section.,. The estimated symbol is associated (330) with a command which is performed (340). Processes for making the symbol estimation are discussed in further detail below. Commands can be any arbitrary command carried out by an output device. For example, if the user is attempting to write, the command for each letter symbol can be associated with the command to append the letter to the current text input string. Non-letter symbols (e.g. a question mark) can be associated with appending that non-letter symbol. In some embodiments, arbitrary symbols are associated with appending a specific non-letter or letter symbol, such as (but not limited to) using a square to append a tilde, or a spiral to insert a period.

Commands are not limited to text input. Indeed, any arbitrary computer function, including highly complex, pre-determined series of instructions, can be associated with a symbol. For example, a drawn cube may be associated with opening a music streaming program and selecting a particular playlist for immediate playback. As can be readily appreciated, the combinations are endless and the association between symbols and commands can be changed at any time as desired. However, making a correct estimation of the desired symbol is a non-trivial task. Further discussion of how to estimate intended symbols is found below.

### Estimating Symbols using Symbol Models

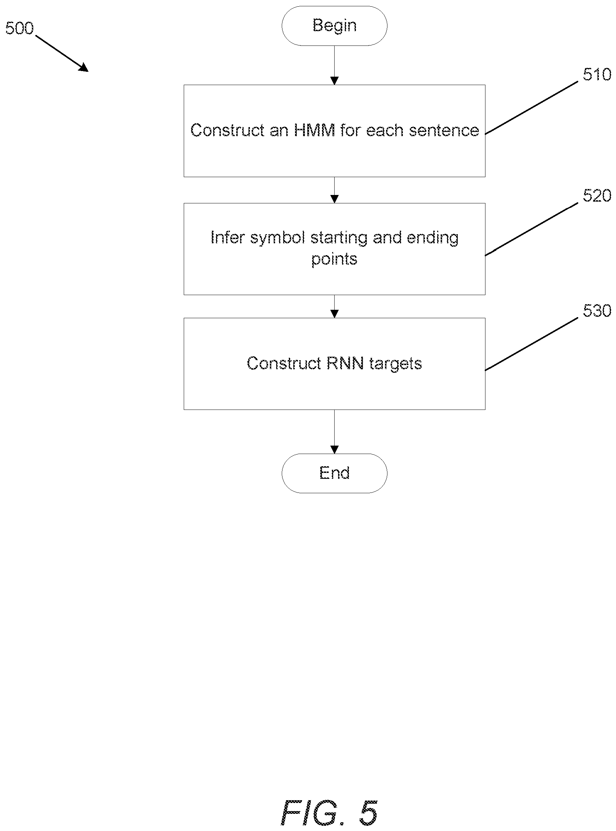

In many embodiments, symbols are estimated using a trained neural network to generate probabilities reflecting the likelihood that a given particular is being "written." In numerous embodiments, the neural network is a recurrent neural network (RNN) which outputs a probability time series describing the likelihood of each character and/or the probability of any new character beginning. However, as noted above, there are many different models that can be used to estimate a symbol from repeatable spike patterns that arise from physical movement that traces a trajectory. For example (long short-term memory) LSTM networks, temporal convolutional networks can be used as well as non-network-based models such as (but not limited to) hidden Markov models (HMMs), which can also infer underlying sequences of symbols from neural recordings. In a variety of embodiments, the neural signal data is preprocessed before being provided to the neural network. A flowchart for a process for estimating intended symbols from neural signal data in accordance with an example is illustrated in FIG. 5

Process 400 includes preprocessing (410) the neural signal data. In many embodiments, the neural signal data is preprocessed by temporally binning and/or temporally smoothing detected spikes on each electrode in the microelectrode array. In many embodiments, the neural signals are analog filtered and digitized. In some embodiments, the analog filter is from 0.3 Hz to 7.5 kHz, and the filtered signals are digitized at 30 kHz at 250 nV resolution. A common average reference filter can be applied to the digitized signals to subtract the average signal across the microelectrode array from every electrode in order to reduce common mode noise. A digital bandpass filter from approximately 250 Hz to 3000 Hz can then be applied. Threshold crossings for each electrode can be performed and the threshold crossing times binned In many embodiments, the threshold crossing is placed at -3.5 x RMS for each electrode, where RMS is the electrode-specific root mean square of the voltage time series recorded for that electrode. In numerous embodiments the temporal binning window is between 10 ms and 300 ms. However, different binning windows can be used based on the user's individual brain. Of note is that each brain is highly idiosyncratic, and many parameters described above and elsewhere can be tuned to produce better results for an individual user. Each bin constitutes a neural population time series referred to as xt.

The neural population time series are provided to an RNN to produce a probability time series. In many embodiments, the RNN is trained to produce a set of probabilities reflective of which symbols are most likely to be associated with the particular population time series. In various embodiments, the RNN is given an output delay value d so the RNN has time to observe multiple population time series which may be associated with the "drawing" of the same symbol. For example, a 1 second output delay would enable 10, 100ms bins to be observed per probability time series. Again, depending on the ability of the user's brain, longer or shorter bins and output delays may be used.

The resulting time probability series (*p_{t-d}*) is thresholded to select (430) the most probable symbol. In numerous embodiments, when the probability for a "new character" crosses a threshold at time t, the most likely character at time t+0.3 is selected. However, depending on the speed of the individual, the positive offset can be adjusted. In some cases, it may take on the order of seconds for a user to imagine handwriting a symbol depending on the symbols available.

In embodiments of the invention, a language model is used to reduce errors. Any number of different natural language processing techniques can be added on the back end. However, the raw output is often sufficient, and adding error correction for natural language can increase delay times due to processing time. In some embodiments, error correction can be manually activated only when appropriate, for example when writing long documents.

As noted above, every brain is idiosyncratic, and therefore a generic RNN may not work for every individual. However, a pre-trained RNN can be calibrated to a particular individual. Further, conventional RNN architectures have not presently shown the ability to recognize intended symbols in neural signals. In order to address this deficiency, systems and methods described herein can utilize specialy architected decoder RNNs. In many embodiments, a gated recurrent RNN is used. In various embodiments, the recurrent RNN is formalized as: ${r}_{t} = a \left({W}_{r}{X}_{t}+{R}_{r}{h}_{t - 1}+{b}_{Wr}+{b}_{Rr}\right)$ ${u}_{t} = a \left({W}_{u}{X}_{t}+{R}_{u}{h}_{t - 1}+{b}_{Wu}+{b}_{Ru}\right)$ ${c}_{t} = {a}_{h} \left({W}_{h}{X}_{t}+{r}_{t}*\left({R}_{h}{h}_{t - 1}+{b}_{Rh}\right)+{b}_{Wh}\right)$ ${h}_{t} = \left(1-{u}_{t}\right) * {c}_{t} + {u}_{t} * {h}_{t - 1}$ where, a is the logistic sigmoid function, *aₕ* is the hyperbolic tangent, *xₜ* is the input vector at time step *t, hₜ* is the hidden state vector, *rₜ* is the reset gate vector, *uₜ* is the update gate vector, *ct is* the candidate hidden state vector, *W, R* and b are parameter matrices and vectors, and * denotes the element-wise multiplication.

In various embodiments, the gated recurrent RNN utilizes two layers, where the hidden state of the first layer is fed as input to the second layer. As noted above, in numerous embodiments the RNN is trained with an output delay. E.g., the RNN can be trained to predict the symbol probabilities from any arbitrary amount of time in the past. The output probabilities can be computed from the hidden state of the second layer using the following: ${y}_{t} = softmax \left({W}_{y}{h}_{t}+{b}_{y}\right)$ ${z}_{t} = a \left({W}_{z}{h}_{t}+{b}_{z}\right)$ where, a is the logistic sigmoid function, *hₜ* is the hidden state of the second layer, *W* and b are parameter matrices and vectors, *yₜ* is a vector of character probabilities (one entry for each symbol), and *zₜ* is a scalar probability that represents the probability of any new symbol beginning at that time step. Whenever *zₜ* crosses a predetermined threshold as described above, the most probable symbol in *yₜ* is emitted after the time delay.

Training RNNs as described herein for symbol decoding systems can be challenging because training data can be difficult to obtain. In many embodiments, training data specific to the user needs to be generated to account for their particular brain. However, many users of symbol decoding systems have some inability to interface with machines using traditional methods (e.g. keyboards, mice, etc.). Therefore it can be difficult to label training data as it is hard or impossible to specifically determine what exactly the user is imagining at a given moment. In numerous embodiments, this can be overcome using forced alignment labeling with hidden Markov Models (HMMs), or using an unsupervised inference method with connectionist temporal classification (and/or other similar cost functions).

In forced alignment methods, an HMM can be used to infer which symbol is being "written" at each time step, fusing knowledge of the sequence of symbols that were supposed to be written with the neural signal data recorded. These symbol labels can then be used to construct target probabilities that the RNN is trained to reproduce. A process for a constructing RNN targets as training data in accordance with an example is illustrated in FIG. 5.

Process 500 includes constructing (510) an HMM for each sentence of symbols (e.g. letters). In many embodiments, neural signal data for a single symbol are averaged to generate "neural templates" for each symbol. The templates can then be used to define the emission probabilities of the sentence HMMs. The sentence HMMs can then be used to infer (520) symbol starting and ending points within the neural signal data. In various embodiments, the Viterbi algorithm is used to find the most probable start time for each character given the neural activity. RNN targets are constructed (530) based on the start times for each character. For example, target time series of symbol probabilities can be constructed for the RNN to reproduce.

The vector of target symbol probabilities (denoted as *yₜ* above) can be constructed by setting the probability at each time step to be a one-hot representation of the most recently started symbol. The scalar symbol start probability (denoted as *zₜ* above) can be set to be equal to 1 for a 200 ms window after each symbol began, and was otherwise equal to 0. However, the window size can be modified as discussed above, and the 200 ms window is provided as an example. The symbol start probability allows the decoder to distinguish repeated characters from single characters (e.g., "oo" vs. "o").

One advantage of this strategy for representing the RNN output is that uncertainty about whether pauses are occurring between symbols should not degrade performance, since the labeling routine only needs to identify when each symbol begins (not when it ends). Note that this representation causes the RNN to output a "sample-and-hold"-type signal, where it will continue to output the most recently started symbol until the next one begins.

Labeled data can be broken down such that the signal associated with each symbol can be separated out and recombined into artificial symbol strings (e.g. artificial sentences) which can be added to training data to augment it and to prevent overfitting. This process can be repeated multiple times with the user providing more data each time to create a more robust training data set. In many embodiments, noise can be added to these "neural templates" to yield a more robust RNN.

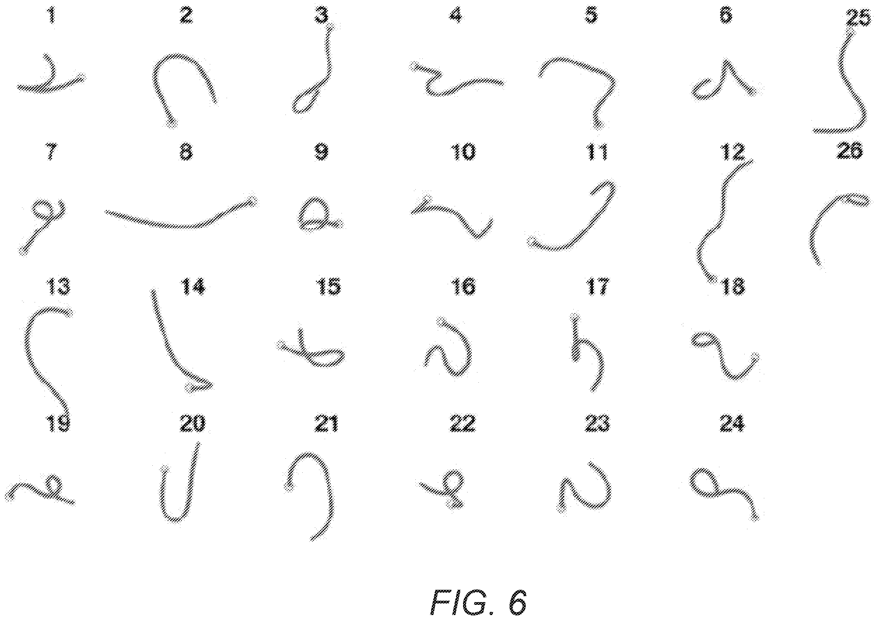

While a particular RNN and training methodology are discussed above, as can be readily appreciated, alternative neural network architectures can be used without departing from the scope of the invention, for example as noted above, LSTMs, temporal convolutional networks, HMMs, and any other model capable of inferring underlying sequences of symbols from neural recordings. Also, when the computation at hand is not required to be causal, the RNN can be made bidirectional. Further, parameters can be modified based on the needs of the individual user. Additionally, training data for each individual user can be made to the needs of the user by generating training data for symbols the user wants to use. However, as noted above, it can be beneficial to select a symbol space that maximizes the difference between each symbol so that the timevarying patterns of movement are more easily discernable by the RNN. An example symbol space consisting of 26 symbols in accordance with an embodiment of the invention is illustrated in FIG. 6.

Although specific systems and methods for decoding intended symbols from neural activity are discussed above, many different system architectures and decoding methods can be implemented in accordance with many different embodiments of the invention. It is therefore to be understood that the present invention may be practiced in ways other than specifically described. Thus, embodiments of the present invention described above should be considered in all respects as illustrative and not restrictive.

## Claims

1. A brain-computer interface for text input, comprising:
a processor (210);
a microelectrode (110) array adapted to be implanted into a brain of a user; and
a memory (230), where the memory comprises a handwriting application capable of directing the processor to:
obtain neural signal data from the microelectrode array, where:
the neural signal data describes neural activity in the user's brain corresponding to handwriting at least one character from an alphabet of characters; and
the distance between a closest pair of characters in the alphabet of characters is maximized;
estimate the at least one character from the neural signal data using a model;
provide the at least one character as a text string; and
use a language model to correct errors in the text string.

2. A method of inputting text using a brain-computer interface, comprising:
obtaining neural signal data from a microelectrode array (110) implanted in a user's brain, where:
the neural signal data describes neural activity in the user's brain corresponding to handwriting at least one character from an alphabet of characters; and
the distance between a closest pair of characters in the alphabet of characters is maximized;
estimating the at least one character from the neural signal data using a model:
providing the at least one character as a text string; and
using a language model to correct errors in the text string.

3. The brain computer interface of claim 1 or method of inputting text using a brain-computer interface of claim 2, wherein the distance between the closest pair of characters is defined by the Frobenius norm of the difference between a trajectory of each character in the pair of characters.

## Patentansprüche

1. Gehirn-Computer-Schnittstelle zur Texteingabe, umfassend:
einen Prozessor (210);
ein Array von Mikroelektroden (110), das dazu ausgelegt ist, in ein Gehirn eines Benutzers implantiert zu werden; und
einen Speicher (230), wobei der Speicher eine Handschriftanwendung umfasst, die in der Lage ist, den Prozessor zu Folgendem anzuweisen:
Erlangen von neuronalen Signaldaten von dem Mikroelektrodenarray, wobei:
die neuronalen Signaldaten eine neuronale Aktivität im Gehirn des Benutzers beschreiben, die einem Schreiben mit der Hand mindestens eines Zeichens aus einem Alphabet von Zeichen entspricht; und
der Abstand zwischen einem nächsten Paar von Zeichen in dem Alphabet von Zeichen maximiert ist;
Schätzen des mindestens einen Zeichens aus den neuronalen Signaldaten unter Verwendung eines Modells;
Bereitstellen des mindestens einen Zeichens als eine Textfolge; und
Verwenden eines Sprachmodells, um Fehler in der Textfolge zu korrigieren.

2. Verfahren zum Eingeben von Text unter Verwendung einer Gehirn-Computer-Schnittstelle, umfassend:
Erlangen neuronaler Signaldaten von einem Mikroelektrodenarray (110), das in dem Gehirn eines Benutzers implantiert ist, wobei:
die neuronalen Signaldaten eine neuronale Aktivität im Gehirn des Benutzers beschreiben, die einem Schreiben mit der Hand mindestens eines Zeichens aus einem Alphabet von Zeichen entspricht; und
der Abstand zwischen einem nächsten Paar von Zeichen in dem Alphabet von Zeichen maximiert ist;
Schätzen des mindestens einen Zeichens aus den neuronalen Signaldaten unter Verwendung eines Modells;
Bereitstellen des mindestens einen Zeichens als eine Textfolge; und
Verwenden eines Sprachmodells, um Fehler in der Textfolge zu korrigieren.

3. Gehirn-Computer-Schnittstelle nach Anspruch 1 oder Verfahren zum Eingeben von Text unter Verwendung einer Gehirn-Computer-Schnittstelle nach Anspruch 2, wobei der Abstand zwischen dem nächsten Paar von Zeichen durch die Frobenius-Norm der Differenz zwischen einer Trajektorie jedes Zeichens in dem Paar von Zeichen definiert ist.

## Revendications

1. Interface cerveau-ordinateur pour l'entrée de texte, comprenant :
un processeur (210) ;
un réseau de microélectrodes (110) adapté pour être implanté dans le cerveau d'un utilisateur ; et
une mémoire (230), où la mémoire comprend une application d'écriture manuscrite capable de diriger le processeur pour :
obtenir des données de signaux neuronaux à partir du réseau de microélectrodes, où :
les données de signaux neuronaux décrivent l'activité neuronale dans le cerveau de l'utilisateur correspondant à l'écriture manuscrite d'au moins un caractère provenant d'un alphabet de caractères ; et
la distance entre une paire de caractères la plus proche dans l'alphabet de caractères étant maximisée ;
estimer l'au moins un caractère à partir des données de signaux neuronaux à l'aide d'un modèle ;
fournir l'au moins un caractère sous forme de chaîne de texte ; et utiliser un modèle de langue pour corriger les erreurs dans la chaîne de texte.

2. Procédé de entrée de texte à l'aide d'une interface cerveau-ordinateur, comprenant :
l'obtention de données de signaux neuronaux à partir d'un réseau de microélectrodes (110) implanté dans le cerveau d'un utilisateur, où :
les données de signaux neuronaux décrivent l'activité neuronale dans le cerveau de l'utilisateur correspondant à l'écriture manuscrite d'au moins un caractère provenant d'un alphabet de caractères ; et
la distance entre une paire de caractères la plus proche dans l'alphabet de caractères étant maximisée ;
l'estimation d'au moins un caractère à partir des données de signaux neuronaux à l'aide d'un modèle fournissant l'au moins un caractère sous forme de chaîne de texte ; et l'utilisation d'un modèle de langue pour corriger les erreurs dans la chaîne de texte.

3. Interface cerveau-ordinateur de la revendication 1 ou procédé d'entrée de texte à l'aide d'une interface cerveau-ordinateur de la revendication 2, ladite distance entre la paire de caractères la plus proche étant définie par la norme de Frobenius de la différence entre une trajectoire de chaque caractère dans la paire de caractères.
